# EUROPÄISCHE PATENTSCHRIFT

(11) **EP 2 664 302 B1**
(45) Veröffentlichungstag und Bekanntmachung des Hinweises auf die Patenterteilung: **11.07.2018**
(21) Anmeldenummer: 13167766.8
(22) Anmeldetag: 15.05.2013
(51) Int. Cl.: A61F 2/58, A61F 2/50, A61F 2/70, A61F 4/00

(54) **Prothese oder Prothesenüberzug**
Prosthesis or prostheses coating
Prothèse ou revêtement de prothèse

(30) Priorität: 15.05.2012 DE 102012009699
(43) Veröffentlichungstag der Anmeldung: 20.11.2013
(73) Patentinhaber: Pohlig GmbH, 83278 Traunstein (DE)
(72) Erfinder: Schäfer, Michael, 83278 Traunstein (DE)
(74) Vertreter: Pfenning, Meinig & Partner mbB

(56) Entgegenhaltungen:
- US-A1- 2009 066 658
- US-A1- 2010 039 392
- US-A1- 2011 265 245
- US-A1- 2011 278 061

## Beschreibung

Die Erfindung betrifft eine Prothese oder einen Prothesenüberzug.

Kapazitiv berührungsempfindliche Eingabegeräte wie beispielsweise Touchscreens reagieren auf Änderungen der Kapazität der berührungsempfindlichen Oberfläche und werden im Allgemeinen mit dem bloßen Finger oder manchmal mit elektrisch leitfähigen Hilfsmitteln wie beispielsweise elektrisch leitfähigen Eingabestiften bedient. Gerade die Bedienung eines Touchscreens mit dem bloßen Finger ermöglicht eine einfache, unmittelbare und direkte Interaktion des Benutzers mit dem auf dem Touchscreen dargestellten Objekt.

Die US 2011/278061 A1 offenbart eine Vorrichtung, die einen distalen Leiter, mit einem ersten, im allgemeinen planaren Substrat, einer Klebeschicht, die auf einer ersten Seite mit dem ersten Substrat verbunden ist, und einem elektrisch leitfähigen Kontakt, der mit dem ersten Substrat auf einer zweiten Seite befestigt ist, einen Konnektor, der elektrisch mit dem Kontakt gekoppelt ist, und einen proximalen Leiter mit einem zweiten, im allgemeinen planaren Substrat, der elektrisch mit dem Konnektor gekoppelt ist, umfasst.

Die US 2010/0039392 A1 offenbart eine Vorrichtung und eine Befestigung an einem Fingernagel eines Benutzers, die eingesetzt werden, um mit einem leitenden Touchscreen zusammenzuwirken. Die Vorrichtung ist insbesondere aus einem leitfähigen Material hergestellt. Die Vorrichtung wird dem Fingernagel des Benutzers verbunden oder auf der Oberseite des Fingers und eines Handschuhs des Benutzers getragen. Wenn der Benutzer den leitenden Touchscreen über die Vorrichtung am Finger berührt, wird eine Störung in der Kapazität erzeugt und dementsprechend die Anwesenheit und/oder die Position des Fingers auf dem Bildschirm erfasst.

Die Bedienung von kapazitiven Eingabegeräten birgt besondere Nachteile für Menschen mit beispielsweise Finger- oder Handprothesen, da einerseits derartige Prothesen im Allgemeinen aus einem isolierenden Material, wie zum Beispiel Silikon, geschaffen sind, welches der Beschaffenheit des zu ersetzenden Körperteils in Konsistenz und Farbe ähnelt. Kapazitive Eingabegeräte lassen sich daher nicht direkt mit der Prothese bedienen. Auch die Verwendung eines elektrisch leitfähigen Eingabestiftes ist mit einer Prothese problematisch, da die zur Positionierung des Eingabestiftes nötigen feinmotorischen Bewegungen meist nicht exakt genug mit der Prothese ausführbar sind.

Ausgehend hiervon ist es daher die Aufgabe der Erfindung eine Prothese oder einen Prothesenüberzug bereitzustellen, welcher die direkte Bedienung eines kapazitiv berührungsempfindlichen Eingabegerätes mit einer hohen Berührungsempfindlichkeit ohne die Verwendung von zusätzlichen Hilfsmitteln wie beispielsweise Eingabestiften ermöglicht und gleichzeltig ermöglicht, die Optik und Beschaffenheit der Prothese oder des Prothesenüberzugs an die Optik und Beschaffenheit eines zu ersetzenden Körperteils anzupassen.

Die Aufgabe wird durch die Prothese oder den Prothesenüberzug nach Anspruch 1 gelöst. Vorteilhafte Weiterbildungen einer erfindungsgemäßen Prothese oder eines erfindungsgemäßen Prothesenüberzugs werden in den abhängigen Ansprüchen 2 bis 12 beschrieben. Die abhängigen Ansprüche 13 und 14 beschreiben Verwendungen der erfindungsgemäßen Prothese oder des erfindungsgemäßen Prothesenüberzugs

Die nachfolgend beschriebene Erfindung ermöglicht es Menschen mit beispielsweise einer Hand- oder Fingerprothese kapazitive Eingabegeräte mit Smart-Technologien wie beispielsweise kapazitive Touchscreens von Mobiltelefonen oder von interaktiven Anzeigetafeln ohne zusätzliche Hilfsmittel unmittelbar zu bedienen.

Die erfindungsgemäße Prothese bzw. der erfindungsgemäße Prothesenüberzug weist dazu mindestens einen Bereich auf, der zur Bedienung eines kapazitiven Eingabegerätes, insbesondere eines kapazitiv berührungsempfindlichen Eingabegerätes, geeignet ist. Unter Bereich wird dabei ein Teil der Prothese verstanden, der in elektrischem und/oder mechanischem Kontakt zur Außenfläche der Prothese steht oder ein Teil der Außenfläche ist und mit Gegenständen aus der Umgebung des Prothesenträgers in elektrischen und/oder mechanischen Kontakt gebracht werden kann.

Des Weiteren weist die Prothese oder der Prothesenüberzug mindestens eine erste Schicht auf, wobei diese Schicht als Bereich zur Bedienung des kapazitiven Eingabegerätes wiederum eine erste Kontaktstelle sowie eine mit der ersten Kontaktstelle elektrisch leitend verbundene zweite Kontaktstelle aufweist. Die erste Kontaktstelle ist dabei mit dem Eingabegerät in Kontakt bringbar. Die zweite Kontaktstelle ist ihrerseits mit einem Körperteil elektrisch leitend verbindbar. Im Folgenden werden die erste und die zweite Kontaktstelle alternativ auch als erste und zweite Kontaktfläche bezeichnet.

Die zweite Kontaktstelle weist hierbei im Allgemeinen eine Fläche von 1 bis 100 mm², vorteilhafterweise von 5 bis 50 mm², vorteilhafterweise von 20 bis 40 mm², vorteilhafterweise von 30 mm² auf. Je größer die Fläche der zweiten Kontaktstelle gewählt wird, desto besser ist der elektrisch leitende Kontakt zwischen der zweiten Kontaktstelle und dem Körperteil. Die maximal mögliche Fläche der zweiten Kontaktstelle wird jedoch beispielsweise durch die Form der Oberfläche des Körperteils, mit der die zweite Kontaktstelle elektrisch leitend verbunden ist, begrenzt.

Die erste und/oder die zweite Kontaktstelle enthalten vorzugsweise leitfähiges Silikon und/oder einen leitfähigen Schaum oder bestehen daraus.

Des Weiteren können die erste und die zweite Kontaktstelle über einen leitfähigen Träger, der Silikon, Polyurethan, Metalle, leitfähiges Garn, ein leitfähiges Kabel, eine leitfähige Flüssigkeit, einen leitfähigen Kanal, oder andere leitfähige Materialien oder Kombinationen hiervon enthält oder daraus besteht, elektrisch leitend miteinander verbunden sein.

Der leitfähige Träger, wie z.B. ein Kabel oder ein leitfähiger Kanal weisen vorteilhafterweise einen Durchschnitt zwischen 1mm und 4mm, besonders bevorzugt zwischen 2mm und 3mm, auf.

In einer bevorzugten Ausführungsform der Erfindung ist der leitfähige Träger Bestandteil der ersten Schicht. Dies ermöglicht eine besonders gesicherte und nachhaltige Positionierung des leitfähigen Trägers, wobei die mechanischen Belastungen, welche z.B. durch während der Verwendung der Prothese oder des Prothesenüberzugs auftretende Biegemomente verursacht werden, reduziert werden.

Des Weiteren weist die Prothese oder der Prothesenüberzug auch mindestens eine innere Schicht als erste Schicht und mindestens eine zweite, äußere Schicht auf, wobei die zweite Schicht die erste Schicht bis auf die Kontaktstellen weitgehend umgibt. Die Kontaktstellen werden also von der zweiten Schicht nicht überdeckt, sodass elektrisch leitende Kontakte zwischen der ersten Kontaktstelle und einem kapazitiven Eingabegerät sowie zwischen der zweiten Kontaktstelle und einem Körperteil herstellbar ist. Die äußere Schicht grenzt somit die innere, erste Schicht bis auf die Kontaktstellen im Wesentlichen gegen die Umgebung, d.h. nach außen, ab. Im Fall eines Prothesenüberzugs sind die äußere und die innere Schicht also derart angeordnet, dass sich die innere Schicht benachbart zur vom Prothesenüberzug aufgenommenen Prothese befindet, und die äußere Schicht im Wesentlichen auf der der Prothese abgewandten Seite der inneren Schicht angeordnet ist. Im Folgenden wird die zweite, äußere Schicht alternativ auch als zweite Schicht oder als äußere Schicht bezeichnet.

Durch die elektrisch leitende Verbindung der ersten Kontaktstelle mit einem großen elektrisch leitfähigen Volumen ist bei einer Kontaktierung der ersten Kontaktstelle mit dem Eingabegerät eine sehr gut messbare Kapazitätserhöhung der kapazitiv berührungsempfindlichen Oberfläche des Eingabegerätes möglich. Dies entspricht einer hohen Berührungsempfindlichkeit des Eingabegerätes. Aufgrund dieser erhöhten Empfindlichkeit ist es wiederum möglich, die erste Kontaktfläche ohne eine wesentliche Verschlechterung der Empfindlichkeit kleiner zu gestalten und so die Positionierungsgenauigkeit der ersten Kontaktfläche auf dem Eingabegerät zur erhöhen.

Aufgrund der elektrisch leitenden Verbindung, die auf diese Weise zwischen einem von einer Prothese aufgenommenen Körperteil und einem kapazitiven Eingabegerät herstellbar ist, kann ein kapazitives Eingabegerät von einem Patienten mit der gleichen Qualität wie mit einem gesunden Körperteil bedient werden.

Die innere Schicht kann in die äußere Schicht einvulkanisiert und/oder eingeklebt sein.

Die zweite Schicht ist vorteilhafterweise elektrisch nichtleitend. Ferner kann die zweite Schicht ein der Haut in Konsistenz und/oder Farbe ähnelndes Material enthalten. Es ist auch denkbar, dass die äußere Schicht Silikon enthält oder zu über 90% daraus besteht. Die erste Schicht kann ebenfalls Silikon enthalten oder zu über 90% daraus bestehen.

Die Elastizität von Silikon ermöglicht ein adaptives Verhalten der Prothese bzw. des Prothesenüberzugs. Ferner wird eine formtreue Gestaltung der Prothese bzw. des Prothesenüberzugs mit freier Farbgestaltung ermöglicht. Durch einen hohen Silikon-Anteil wird die Prothese bzw. der Prothesenüberzug mechanisch hochwertig und erhält einen hohen Tragekomfort.

In einer alternativen Ausführungsform der Erfindung weist die Prothese oder der Prothesenüberzug als Bereich zur Bedienung eines kapazitiven Eingabegerätes eine Kontaktstelle auf, die im Folgenden alternativ auch als Kontaktfläche bezeichnet wird, welche mit einer Eingabeeinheit elektrisch leitend verbunden ist. Die Eingabeeinheit ist vorzugsweise mittels einer klebenden Verbindung an der Prothese oder dem Prothesenüberzug befestigt. Alternativ kann die Eingabeeinheit auch mittels einer Stulpe an der Prothese oder an dem Prothesenüberzug angebracht sein. Ferner ist es denkbar, die Eingabeeinheit mittels Einvulkanisation an der Prothese oder an dem Prothesenüberzug anzubringen.

Die Eingabeeinheit kann auch stiftförmig ausgebildet sein.

Vorzugsweise enthält die Eingabeeinheit und/oder die Kontaktstelle leitfähiges Silikon und/oder einen leitfähigen Schaum oder besteht daraus. Der Bereich zur Bedienung des kapazitiven Eingabegerätes weist vorzugsweise eine Fläche von 1 bis 100 mm², vorteilhafterweise von 5 bis 50 mm², vorteilhafterweise von 20 bis 30 mm², vorteilhafterweise von 25 mm² auf. Hierbei ist eine kleinere Fläche besonders vorteilhaft, da dadurch die Positionierungsgenauigkeit auf einem berührungsempfindlichen Eingabegerät erhöht wird.

Die Prothese oder der Prothesenüberzug enthält hochtemperaturvernetztes Silikon als Basismaterial oder besteht daraus. Ferner kann der Bereich zur Bedienung des kapazitiven Eingabegerätes mit einem dünnen, gegebenenfalls farbadaptierten, Material zum Schutz des Bereichs überzogen sein.

Die oben beschriebene erfindungsgemäße Prothese oder der oben beschriebene erfindungsgemäße Prothesenüberzug ist zur Bedienung eines Touchscreens, Touchdisplays, Touchpads, einer Touchmaus und/oder einer Multi-Touch-Oberfläche geeignet und kann als Finger-, Hand-, Partialhand-, Arm-, Fuß-, Bein-oder Zehenprothese bzw, als Überzug für eine Finger-, Hand-, Partialhand-, Arm-, Fuß-, Bein-, Zehenprothese oder eine Arbeitsprothesenhand, wie z.B. eine Hook-Prothese, ausgebildet sein, wobei die zweite Kontaktstelle mit einem Finger-, Hand-, Arm-, Bein-, Fuß- oder Zehenstumpf elektrisch leitend verbunden ist.

Bei den hier verwendeten Eingabeeinheiten handelt es sich um kapazitivwirkende Eingabeeinheiten. Bringt man die Kontaktfläche der Eingabeeinheit mit beispielsweise einem kapazitiven Touchdisplay in Kontakt, ändert sich an der Kontaktposition, also die Position des Touchdisplays, welche mit der Kontaktfläche in Kontakt steht die Kapazität im Touchdisplay. Die Kontaktposition, an der sich die Kapazität ändert, ist dann über eine Strom- und/oder Spannungsmessung bestimmbar.

Im Folgenden werden einige Ausführungsbeispiele einer erfindungsgemäßen Prothese oder eines erfindungsgemäßen Prothesenüberzugs gegeben. Die gezeigten Merkmale können dabei auch unabhängig von dem konkreten Ausführungsbeispiel realisiert sein. Demnach ist es auch möglich, alle oder einzelne Merkmale der gezeigten Ausführungsbeispiele auf andere Weise zu weiteren Ausführungsbeispielen zu kombinieren. Es zeigen:
Figuren 1 bis 7 Beispiele für Prothesen, welche nicht Teil der Erfindung sind mit einer Kontaktstelle zur Bedienung eines kapazitiv berührungsempfindlichen Eingabegerätes. Die in den Figuren 1 bis 7 gezeigten Prothesen sind nicht Teil der Erfindung. Die Figuren 1 bis 7 und die zugehörige Beschreibung helfen jedoch, die verschiedenen Aspekte der Erfindung besser zu verstehen.
Figuren 8 bis 14 zeigen erfindungsgemäße Prothesen gemäß einem zweiten Ausführungsbeispiel mit einer elektrisch leitenden Verbindung zwischen dem aufgenommenen Körperteil und der ersten Kontaktstelle, darunter zeigen

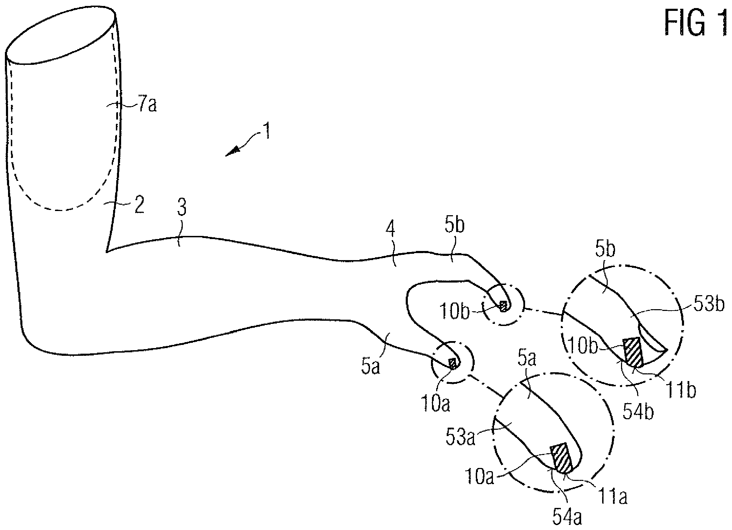

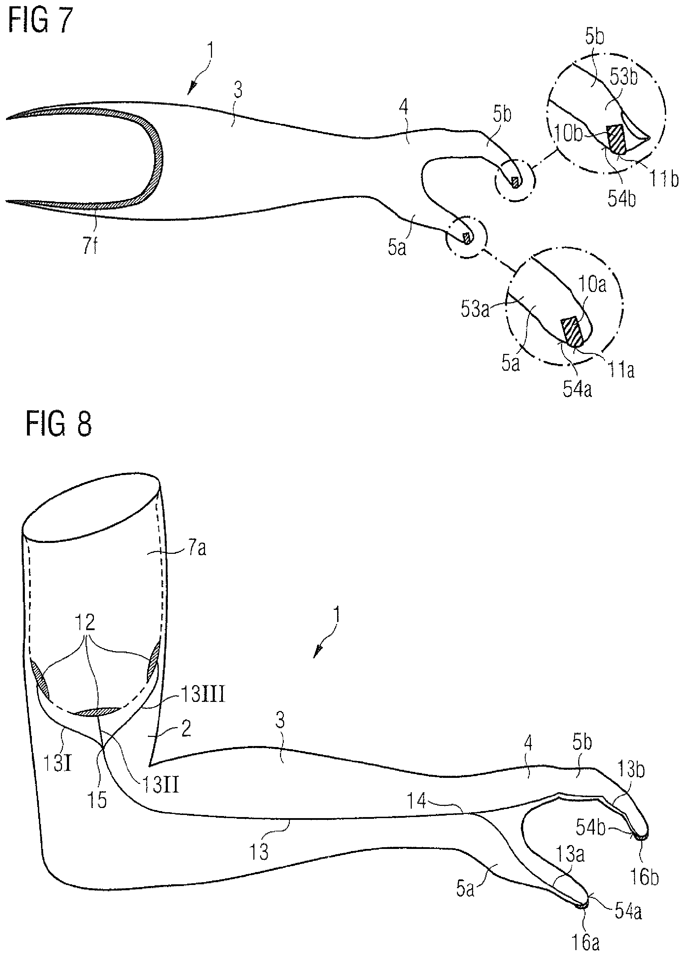

Figuren 1, 8 eine Ellenbogenexartikulations- und Oberarmprothese,

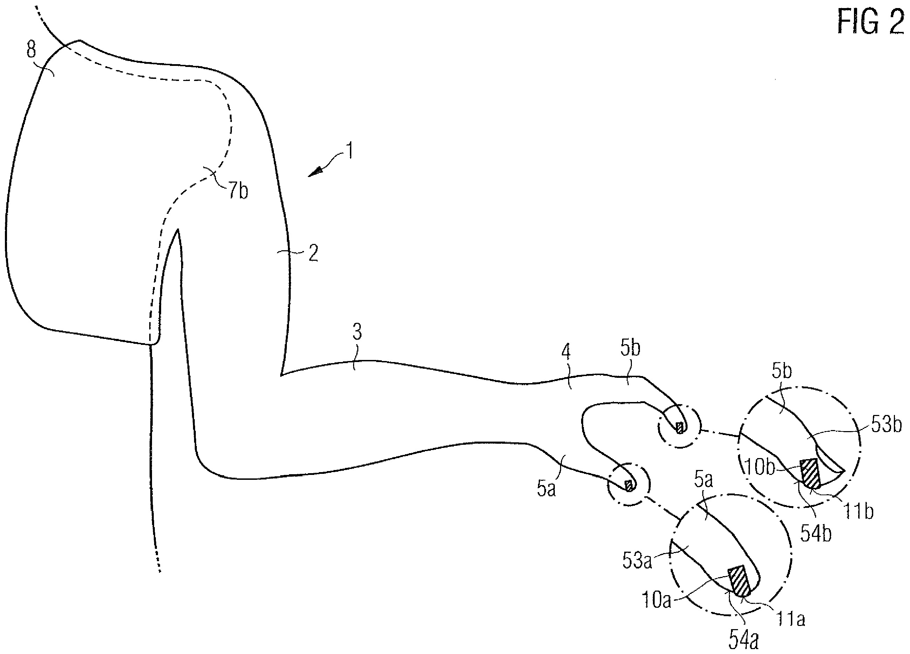

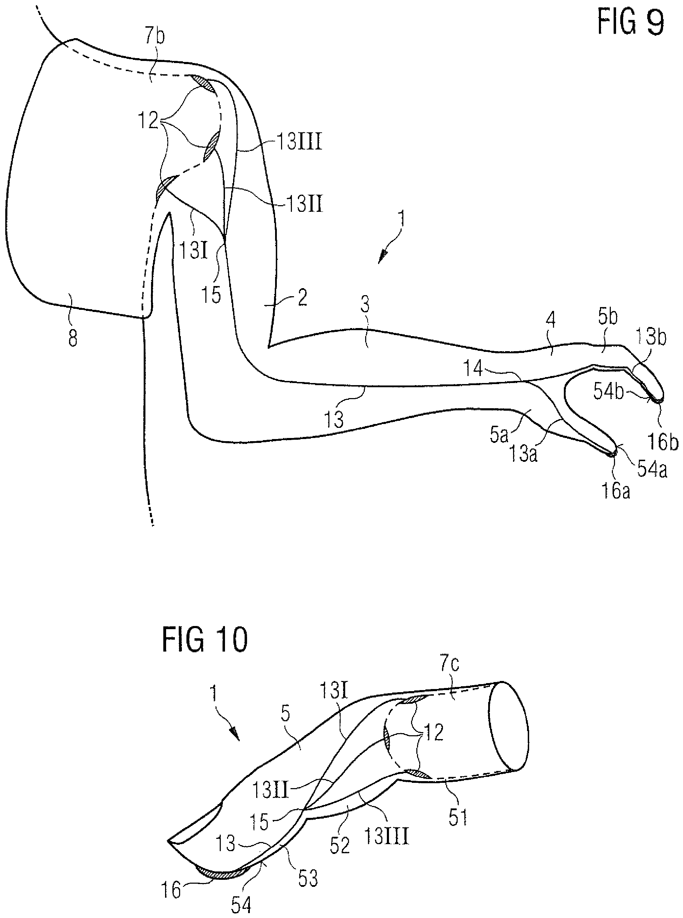

Figuren 2, 9 eine Schulterexartikulations- und Schultergürtelprothese,

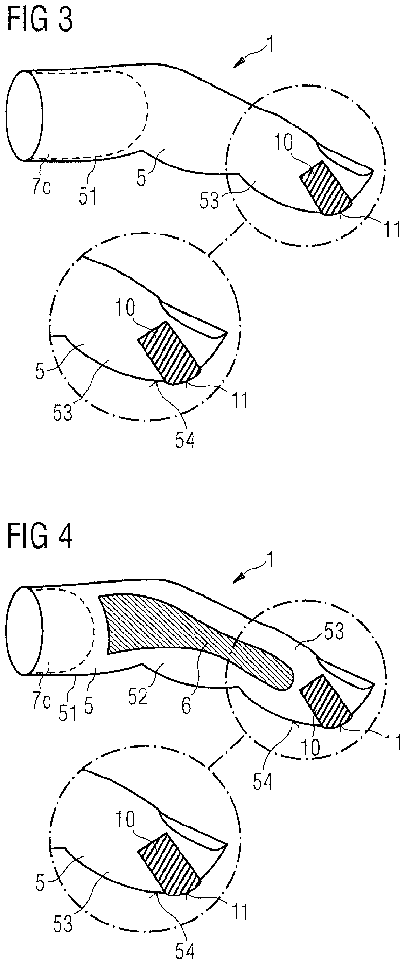

Figuren 3, 10 eine Fingerprothese,

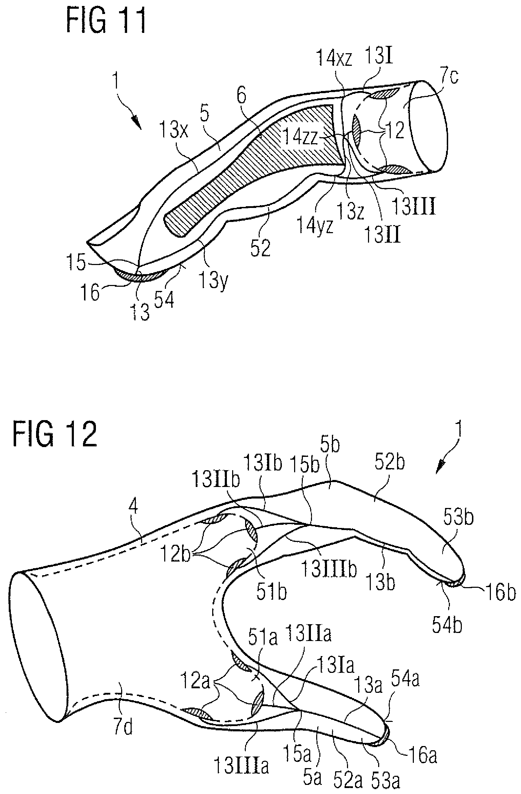

Figuren 4, 11 eine weitere Fingerprothese,

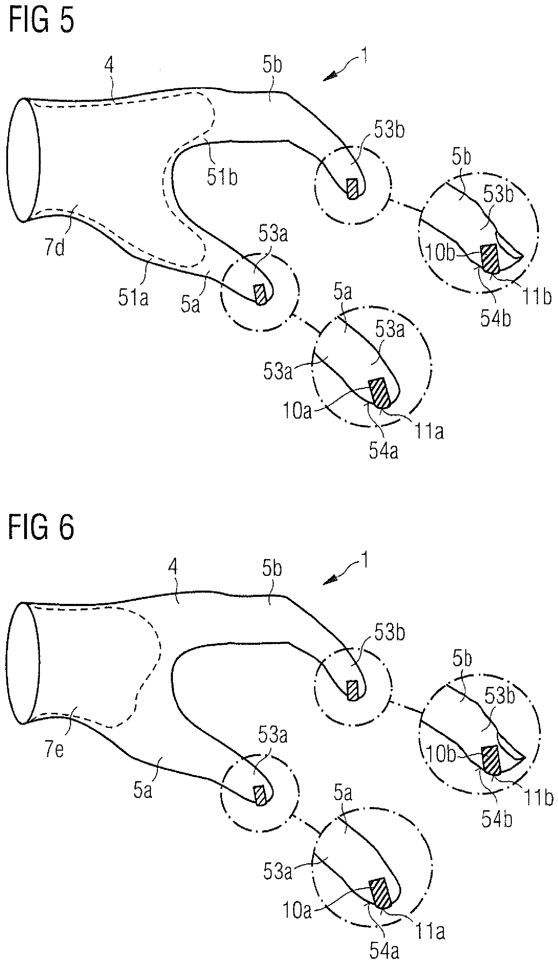

Figuren 5, 12 eine Partialhandprothese,

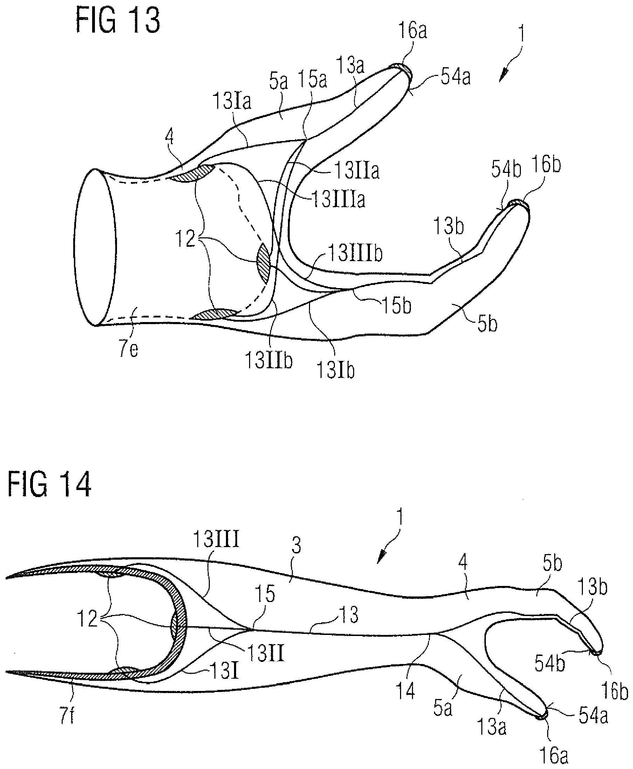

Figuren 6, 13 eine Handprothese,
Figuren 7, 14 eine Unterarmprothese,

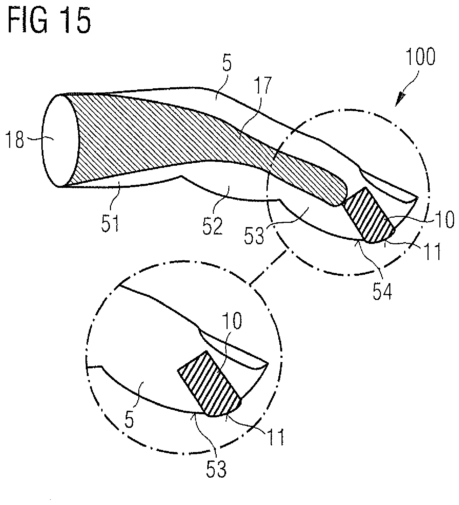

Figur 15 zeigt ein Beispiel für einen Prothesenüberzug. Der in Figur 15 gezeigte Prothesenüberzug ist nicht Teil der Erfindung. Die Figur 15 sowie die zugehörige Beschreibung helfen jedoch, die verschiedenen Aspekte der Erfindung besser zu verstehen.

In der nachfolgenden Beschreibung der Figuren bezeichnen gleiche oder ähnliche Bezugszeichen gleiche oder ähnliche Elemente.

Figur 1 zeigt eine Ellenbogenexartikulations- und/oder Oberarmprothese 1 zur Bedienung eines kapazitiv berührungsempfindlichen Eingabegerätes gemäß einem ersten Ausführungsbeispiel. Die Prothese 1 weist einen Oberarmteil 2, einen Unterarmteil 3 und einen Handteil 4 auf. Ferner weist der Oberarmteil einen Prothesenschaft 7a mit einer zur gedachten Schulter hin offenen, in etwa zylinderförmigen Aushöhlung auf, welche je nach Länge des Amputationsstumpfes bis maximal an das prothetische Ellenbogengelenk heranreicht. Der Prothesenschaft 7a ist geeignet einen menschlichen Oberarmstumpf weitgehend formschlüssig aufzunehmen. Des Weiteren weist der Handteil 4 der Prothese 1 einen künstlichen Daumen 5a und künstliche Finger 5b auf, wobei hier zur Vereinfachung lediglich ein Zeigefinger dargestellt ist. Der Daumen 5a sowie die Finger 5b sind an ihren dem Handteil 4 abgewandten Spitzen im Bereich der Daumen- bzw. Fingerdruckflächen 54a und 54b mit Kontaktflächen 11a bzw. 11b versehen. An einer dem Finger 5b bzw. Daumen 5a abgewandten Seite der Kontaktfläche 11b bzw. 11a befinden sich jeweils Eingabeeinheiten 10b bzw. 10a, die mit den Kontaktflächen 11b bzw. 11a in elektrisch leitendem Kontakt stehen. Die Eingabeeinheiten 10b bzw. 10a sind vorzugsweise von den Kontaktflächen 11b bzw. 11a weg weisend verjüngend ausgebildet. Zur Bedienung eines kapazitiv berührungsempfindlichen Eingabegerätes können die Eingabeeinheiten 10a und 10b mit einer Oberfläche des Eingabegerätes in elektrisch leitfähigen Kontakt gebracht werden. Je geringer dabei die Auflagefläche der Eingabeeinheit ist, desto präziser kann das Eingabegerät bedient werden. Es ist ebenfalls denkbar, nur einzelne Finger 5b oder nur den Daumen 5a mit Kontaktflächen 11b bzw. 11a auszustatten. Je nachdem wie viele Finger als Eingabeeinheiten 10a, 10b fungieren, sind verschiedene Arten von Multi-Touch-Befehien auf Multi-Touch-fähigen Eingabegeräten möglich.

Figur 2 zeigt eine Schulterexartikulations- und Schultergürtelprothese 1 gemäß einem ersten Ausführungsbeispiel. Die Prothese 1 umfasst einen Schulterteil 8, einen Oberarmteil 2, einen Unterarmteil 3 und einen Handteil 4. Der Schulterteil 8 ist weitgehend als Prothesenschaft 7b ausgebildet, der zur Aufnahme eines menschlichen Schulterstumpfes geeignet ist. Die Gestaltung des Prothesenschaftes 7b kann flächig oder in Rahmenkonstruktion erfolgen. Der Schulterteil 8 bedeckt die menschliche Schulter in etwa bis zum Halsansatz. An den Schulterteil 8 schließt sich der Oberarmteil 2 der Prothese 1 an. Der Unterarmteil 3 sowie der Handteil 4 sind wie in Figur 1 beschrieben aufgebaut. Der Handteil 4 weist einen künstlichen Daumen 5a und künstliche Finger 5b auf, wobei hier zur Vereinfachung lediglich ein Zeigefinger dargestellt ist. Der Daumen 5a und die Finger 5b weisen an ihren dem Handteil 4 abgewandten Enden Fingerendglieder 53a bzw. 53b auf. An diesen Fingerendgliedern 53a und 53b sind der Daumen 5a und die Finger 5b im Bereich der Daumen- bzw. Fingerdruckflächen 54a und 54b mit Kontaktflächen 11a bzw. 11b ausgestattet, die an ihren dem Daumen 5a bzw. den Fingern 5b abgewandten Seiten Eingabeeinheiten 10a bzw, 10b aufweisen. Die Eingabeeinheiten 10a und 10b sind mit den Kontaktflächen 11a bzw. 11b elektrisch leitend verbunden. Die Eingabeeinheiten 10a und 10b sind vorteilhafterweise vom Daumen 5a bzw. von den Fingern 5b weg weisend verjüngend ausgebildet. Zur Bedienung eines kapazitiv berührungsempfindlichen Eingabegerätes werden die Eingabeeinheiten 10a und 10b mit einer Oberfläche des Eingabegerätes in elektrisch leitenden Kontakt gebracht. Je geringer dabei die Auflagefläche der Eingabeeinheit ist, desto präziser kann das Eingabegerät bedient werden. Wie auch in Figur 1 ist es hier ebenfalls denkbar nur den Daumen 5a und/oder einzelne Finger 5b mit Kontaktflächen 11a bzw. 11b auszustatten.

Figur 3 zeigt eine Fingerprothese 1 gemäß einem ersten Ausführungsbeispiel in Form eines künstlichen Fingers 5 mit einem ersten Fingerglied 51, einem zweiten Fingerglied 52 und einem Fingerendglied 53. Die Fingerprothese weist einen Prothesenschaft 7c auf, welcher eine in etwa zylinderförmige Aushöhlung aufweist, die zum Ansatz des künstlichen Fingers 5 hin offen ist und sich entlang des ersten künstlichen Fingergliedes 51 erstreckt. Der Prothesenschaft 7c ist zur passgenauen Aufnahme eines menschlichen Fingerstumpfes geeignet. An einem Fingerendglied 53 des künstlichen Fingers 5, also in etwa an der Fingerspitze, ist wie bei den Prothesen der Figuren 1 und 2 im Bereich der Fingerdruckfläche 54 eine Kontaktfläche 11 angeordnet, die an ihrer dem Finger 5 abgewandten Seite eine Eingabeeinheit 10 aufweist. Die Eingabeeinheit 10 ist mit der Kontaktfläche 11 elektrisch leitend verbunden. Die Eingabeeinheit 10 kann somit zur Bedienung eines kapazitiv berührungsempfindlichen Eingabegerätes verwendet werden, wobei zwischen der Eingabeeinheit 10 und einer Oberfläche des Eingabegerätes ein elektrisch leitender Kontakt hergestellt wird.

Figur 4 zeigt eine weitere Variante einer Fingerprothese 1 gemäß einem ersten Ausführungsbeispiel. Diese ist weitgehend wie die Fingerprothese in Figur 3 aufgebaut. Im Gegensatz zur Fingerprothese in Figur 3 weist die Fingerprothese 1 in Figur 4 im Inneren einen Hohlraum 6 auf, der sich über das gesamte zweite Fingerglied 52 des künstlichen Fingers 5 erstreckt und von einem zweiten Fingerglied 52 aus gesehen bis in etwa zu einem Drittel der Länge des ersten Fingergliedes 51 bzw. des Fingerendgliedes_53 jeweils in das erste Fingerglied 51 und das Fingerendglied 53 hineinragt.

Figur 5 zeigt eine Partialhandprothese 1 gemäß einem ersten Ausführungsbeispiel. Die Prothese 1 weist einen Handteil 4, einen Daumen 5a und Finger 5b auf, wobei hier zur Vereinfachung lediglich ein Zeigefinger dargestellt ist. Der Handteil 4 sowie die daran ansetzenden Formbettungen noch vorhandener Fingerstümpfe, wie hier die ersten Fingerglieder 51a und 51b des Daumens 5a bzw. der Finger 5b, bilden einen Prothesenschaft 7d, der zur passgenauen Aufnahme einer Hand mit Fingerstümpfen bzw. noch vorhandenen einzelnen Finger geeignet ist. Wie schon in den vorhergehenden Figuren gezeigt und im zugehörigen Text beschrieben sind die Fingerendglieder 53a und 53b mit Kontaktflächen 11a und 11b ausgestattet, an deren dem Daumen 5a bzw. den Fingern 5b abgewandten Seiten Eingabeeinheiten 10a bzw. 10b angeordnet sind. Zur Bedienung eines kapazitiv berührungsempfindlichen Eingabegerätes können die Eingabeeinheiten 10a und 10b mit einer Oberfläche des Eingabegerätes in elektrisch leitenden Kontakt gebracht werden. Wie bei den Ausführungsformen der vorhergehenden Figuren ist es auch hier möglich lediglich den Daumen 5a und/oder einzelne Finger 5b mit Kontaktflächen auszustatten.

Figur 6 zeigt eine Handprothese 1 gemäß einem ersten Ausführungsbeispiel mit einem Handteil 4, einem künstlichen Daumen 5a und künstlichen Fingern 5b. Der Handteil 4 ist als Prothesenschaft 7e mit einer zum gedachten Unterarm hin offenen, in etwa zylinderförmigen Aushöhlung ausgebildet, welche sich bis zum Ansatz des künstlichen Daumens 5a und der künstlichen Finger 5b erstreckt. Der Prothesenschaft 7e ist anders als der Prothesenschaft 7d in Figur 5 zur passgenauen Aufnahme eines transcarpalen und Handstumpfes ohne Fingerstümpfe geeignet. Wie schon in den vorhergehenden Figuren sind der Daumen 5a und die Finger 5b im Bereich der Fingerdruckflächen 54a bzw. 54b der Fingerendglieder 53a bzw. 53b mit Kontaktflächen ausgestattet, die ihrerseits die Eingabeeinheiten 10a und 10b aufweisen. Wie bei den Ausführungsformen der vorhergehenden Figuren ist es auch hier möglich lediglich den Daumen 5a und/oder einzelne Finger 5b mit Kontaktflächen auszustatten.

Figur 7 zeigt eine Unterarmprothese 1 gemäß einem ersten Ausführungsbeispiel mit einem Unterarmteil 3, einem Handteil 4 sowie künstlichen Fingern 5a und 5b, wobei hier zur Vereinfachung lediglich der Daumen 5a und der Zeigefinger 5b dargestellt sind. Der Unterarmteil weist einen Kondylen-freien oder Kondylus-umgreifenden Prothesenschaft 7f auf, der sich von einem gedachten Ellenbogengelenk entlang in etwa der Hälfte des Unterarmteils erstreckt. Der Prothesenschaft 7f ist zur passgenauen Aufnahme eines Unterarmstumpfes geeignet. An den Daumen 5a sowie an die Finger 5b sind wie bei den Ausführungsformen der vorangehenden Figuren Kontaktflächen 11a bzw. 11b mit Eingabeeinheiten 10a bzw. 10b angeordnet, wobei die Eingabeeinheiten 10a und 10b mit den Kontaktflächen in elektrisch leitendem Kontakt stehen. Zur Bedienung eines kapazitiv berührungsempfindlichen Eingabegerätes können die Eingabeeinheiten 10a und 10b mit einer Oberfläche des Eingabegerätes in elektrisch leitenden Kontakt gebracht werden. Wie bei den Ausführungsformen der vorhergehenden Figuren ist es auch hier möglich lediglich den Daumen 5a und/oder einzelne Finger 5b mit Kontaktflächen 11a bzw. 11b und Eingabeeinheiten 10a bzw. 10b auszustatten.

Figur 8 zeigt eine erfindungsgemäße Ellenbogenexartikulations- und/oder Oberarmprothese 1 wie in Figur 1 gemäß einem zweiten Ausführungsbeispiel. Im Unterschied zu Figur 1 sind der Daumen 5a und die Finger 5b an ihren dem Handteil 4 abgewandten Spitzen im Bereich der Daumen- bzw. Fingerdruckflächen 54a und 54b mit ersten Kontaktflächen 16a bzw. 16b ausgestattet. Die Kontaktflächen 16a und 16b sind auf ihrer von der Daumen- bzw. Fingerspitze abgewandten Seite verjüngend oder halbkugelförmig ausgebildet. Die ersten Kontaktflächen 16a bzw. 16b sind jeweils elektrisch leitend mit elektrisch leitenden Kanälen 13a und 13b verbunden, die sich jeweils von den ersten Kontaktflächen 16a und 16b entlang des Daumens 5a bzw. der Finger 5b in den Handteil 4 erstrecken und im Handteil 4 in einer Mündung 14 zu einem elektrisch leitfähigen Kanal 13 zusammenlaufen. Der Kanal 13 erstreckt sich von der Mündung 14 entlang des Unterarmteils 13 bis hin zu einem Verzweigungspunkt 15 im Bereich des prothetischen Ellenbogengelenks. Im Verzweigungspunkt 15 teilt sich der Kanal 13 in drei elektrisch leitfähige Kanäle 13I, 13II, 13III auf, welche sich entlang des Oberarmteils 2 bis zum unteren, dem prothetischen Ellenbogengelenk zugewandten Bereich des Prothesenschafts 7a erstrecken. In dem unteren Bereich des Prothesenschafts 7a sind drei in etwa maximal zueinander beabstandete zweite Kontaktflächen 12 angeordnet, die jeweils mit einem der drei sich vom Verzweigungspunkt 15 erstreckenden Kanäle 13I, 13II und 13III elektrisch leitfähig verbunden sind. Die zweiten Kontaktflächen 12 sind ferner derart am Prothesenschaft 7a angeordnet, dass diese während des Tragens der Prothese 1 in elektrisch leitfähigem Kontakt mit einem Oberarmstumpf stehen. Somit besteht während des Tragens der Prothese 1 zwischen den ersten Kontaktflächen 16a und 16b und einem Oberarmstumpf über die Kanäle 13a bzw. 13b, 13 und 13I, 13II, 13III und die zweiten Kontaktflächen 12 ein elektrischer Kontakt.

Figur 9 zeigt eine erfindungsgemäße Schulterexartikulations- und Schultergürtelprothese 1 wie in Figur 2 gemäß einem zweiten Ausführungsbeispiel. Der Handteil 4 sowie der Unterarmteil 3 sind wie in Figur 8 aufgebaut. Im Unterschied zur Prothese in Figur 8 erstreckt sich der elektrisch leitfähige Kanal 13 weiter in dem Oberarmteil 2 hinein, so dass sich der Verzweigungspunkt 15 im an die Schulter angrenzen Bereich des Oberarmteils 2 befindet. Im Verzweigungspunkt 15 teilt sich der Kanal 13 in drei Kanäle 13I, 13 II und 13 III auf, welche sich wiederum bis zum Prothesenschaft 7b erstrecken. Im an den Oberarmteil 2 angrenzen Bereich des Prothesenschafts 7b, also im Bereich des prothetischen Schultergelenks, sind drei zweite Kontaktflächen 12 in etwa maximal zueinander beabstandet angeordnet. Jede dieser drei zweiten Kontaktflächen 12 ist jeweils mit einem der Kanäle 13I, 13II und 13III elektrisch leitfähig verbunden. Die zweiten Kontaktflächen 12 sind ferner derart am Prothesenschaft 7b angeordnet, dass diese während des Tragens der Prothese 1 in elektrisch leitfähigem Kontakt mit einem Schulterstumpf stehen. Somit besteht während des Tragens der Prothese 1 zwischen den ersten Kontaktflächen 16a und 16b und einem Schulterstumpf über die Kanäle 13a bzw. 13b, 13 und 13I, 13II, 13III und die zweiten Kontaktflächen 12 ein elektrischer Kontakt.

Figur 10 zeigt eine erfindungsgemäße Fingerprothese 1 wie in Figur 3 gemäß einem zweiten Ausführungsbeispiel. Im Unterschied zur Fingerprothese in Figur 3 weist die Fingerprothese 1 in Figur 10 im Bereich der Fingerdruckfläche 54 eine erste Kontaktfläche 16 auf, die elektrisch leitend mit einem elektrisch leitenden Kanal 13 verbunden ist. Die Kontaktfläche 15 ist auf ihrer von der Fingerspitze abgewandten Seite verjüngend oder halbkugelförmig ausgebildet. Der Kanal 13 erstreckt sich von der ersten Kontaktfläche 16 entlang des Fingerendgliedes 53 bis zu einem Verzweigungspunkt 15 im Bereich zwischen dem zweiten Fingerglied 52 und dem Fingerendglied 53. Im Verzweigungspunkt 15 teilt sich der Kanal 13 in drei Kanäle 13I, 13II und 13 III auf, die sich vom Verzweigungspunkt 15 entlang des zweiten Fingergliedes 52 bis zum Prothesenschaft 7c. Im dem zweiten Fingerglied 52 zugewandten Bereich des Prothesenschaftes 7c sind drei zweite Kontaktflächen 12 in etwa maximal zueinander beabstandet angeordnet und jeweils mit einem der Kanäle 13I, 13II und 13III elektrisch leitend verbunden. Auch hier sind die zweiten Kontaktflächen 12 derart am Prothesenschaft 7c angeordnet, dass die zweiten Kontaktflächen 12 beim Tragen der Prothese 1 in elektrisch leitendem Kontakt mit einem Fingerstumpf stehen. Somit besteht während des Tragens der Prothese 1 zwischen der ersten Kontaktfläche 16 und einem Fingerstumpf über die Kanäle 13, 13I, 13II und 13III und die zweiten Kontaktflächen 12 ein elektrischer Kontakt.

Figur 11 zeigt eine weitere erfindungsgemäße Fingerprothese 1 wie in Figur 4 gemäß einem zweiten Ausführungsbeispiel. Wie die Fingerprothese in Figur 10 ist auch die Fingerprothese in Figur 11 im Bereich der Fingerdruckfläche 54 mit einer ersten Kontaktfläche 16 versehen. Diese ist mit einem elektrisch leitenden Kanal 13 elektrisch leitend verbunden. Auf der der Fingerspitze abgewandten Seite der ersten Kontaktfläche 16 befindet sich ein Verzweigungspunkt 15, in dem sich der Kanal 13 in zwei elektrisch leitfähige Kanäle 13x und 13y aufspaltet. Der Kanal 13x erstreckt sich dabei oberhalb des Hohlraums 6, also im Bereich des Fingerrückens, entlang des Fingerendgliedes 53 sowie des zweiten Fingergliedes 52 bis zu einer Mündung 14xz in etwa im Bereich der Hälfte des ersten Fingergliedes 51. Der Kanal 13y erstreckt sich unterhalb des Hohlraums 6, also im Bereich der Innenfläche des Fingers entlang des Fingerendgliedes 53 sowie des zweiten Fingergliedes 52 bis zu einer Mündung 14yz in etwa im Bereich der Hälfte des ersten Fingergliedes 51. Die Mündungen 14xz und 14yz sind auf der dem Prothesenschaft 7c zugewandten Seite durch einen Kanal 13z elektrisch leitend miteinander verbunden. Von der Mündung 14xz erstreckt sich ein Kanal 13I bis zum Prothesenschaft 7c, von der Mündung 14yz erstreckt sich ein Kanal 13 III bis zum Prothesenschaft 7c und von einer sich auf mittlerer Länge des Kanals 13z befindlichen Mündung 14zz erstreckt sich der Kanal 13III bis zum Prothesenschaft 7c. Im dem Hohlraum 6 zugewandten Bereich des Prothesenschafts 7c sind drei zweite Kontaktflächen 12 in etwa maximal zueinander beabstandet angeordnet. Jede der drei zweiten Kontaktflächen 12 ist jeweils mit einem der Kanäle 13I, 13II und 13III elektrisch leitend verbunden. Auch hier sind die zweiten Kontaktflächen 12 derart am Prothesenschaft 7c angeordnet, dass während des Tragens der Prothese 1 ein Fingerstumpf in elektrischem Kontakt mit den zweiten Kontaktflächen 12 steht. Somit besteht während des Tragens der Prothese 1 zwischen der ersten Kontaktfläche 16 und einem Fingerstumpf über die Kanäle 13, 13x, 13y, 13z, 13I, 13II und 13III und die zweiten Kontaktflächen 12 ein elektrischer Kontakt.

Figur 12 zeigt eine erfindungsgemäße Partialhandprothese 1 wie in Figur 5 gemäß einem zweiten Ausführungsbeispiel. Im Unterschied zur Prothese in Figur 5 weist die Prothese 1 in Figur 12 im Bereich der Daumen- und Fingerdruckflächen 54a bzw. 54b erste Kontaktflächen 16a bzw. 16b auf. Die ersten Kontaktflächen 16a und 16b sind jeweils mit einem elektrisch leitenden Kanal 13a bzw. 13b elektrisch leitend verbunden. Die Kanäle 13a und 13b erstrecken sich jeweils von den ersten Kontaktflächen 16a bzw. 16b entlang der Fingerendglieder 53a bzw. 53b sowie entlang der zweiten 52a bzw. 52b und ersten Fingerglieder 51a bzw. 51b bis zu Verzweigungspunkten 15a und 15b in den mittleren Bereichen der ersten Fingerglieder 51a und 51. In den Verzweigungspunkten 15a und 15b teilen sich die Kanäle 13a und 13b jeweils in drei Kanäle 13Ia, 13IIa, 13IIIa und 13Ib, 13IIb, 13IIIb, die sich bis wiederum bis zu den jeweils den Fingern 5b und dem Daumen 5a zugewandten Bereichen des Prothesenschaftes 7d erstrecken. An den den Fingern 5b und dem Daumen 5a zugewandten Bereichen des Prothesenschaftes 7d sind jeweils drei zweite Kontaktflächen 12a bzw. 12b angeordnet. Die zweiten Kontaktflächen 12a sind mit jeweils einem der Kanäle 13Ia, 13IIa und 13IIIa elektrisch leitend verbunden. Die zweiten Kontaktflächen 12b sind mit jeweils einem der Kanäle 13Ib, 13IIb und 13IIIb elektrisch leitend verbunden. Auch hier sind die zweiten Kontaktflächen 12a und 12b derart am Prothesenschaft 7d angeordnet, dass während des Tragens der Prothese 1 Fingerstümpfe in elektrischem Kontakt mit den zweiten Kontaktflächen 12a und 12b stehen. Somit besteht während des Tragens der Prothese 1 zwischen der ersten Kontaktfläche 16a und einem Daumenstumpf über die Kanäle 13a, 13Ia, 13IIa und 13IIIa sowie die zweiten Kontaktflächen 12a ein elektrischer Kontakt.

Figur 13 zeigt eine erfindungsgemäße Handprothese wie in Figur 6 gemäß einem zweiten Ausführungsbeispiels. Im Unterschied zur Prothese in Figur 12 ist die Handprothese 1 in Figur 13 zur Aufnahme eines Mittelhandstumpfes geeignet. Im den Fingern 5a und 5b zugewandten Bereich des Prothesenschaftes 7e sind zweite Kontaktflächen 12 in etwa maximal beabstandet zueinander derart angeordnet, dass ein Mittelhandstumpf während des Tragens der Prothese 1 in elektrisch leitendem Kontakt mit den zweiten Kontaktflächen 12 steht. Wie die Prothese in Figur 12 weist die Prothese 1 in Figur 13 im Bereich der Daumen- bzw. Fingerdruckflächen 54a und 54b erste Kontaktflächen 16a und 16b auf die jeweils mit einem elektrisch leitenden Kanal 13a bzw. 13b elektrisch leitend verbunden sind. Die Kanäle 13a und 13b erstrecken sich jeweils entlang der Fingerendglieder 13a und 13b sowie entlang der zweiten 52a, 52b und ersten Fingerglieder 51a und 51b bis zu Verzweigungspunkten 15a bzw. 15b, in denen sich die Kanäle 13a und 13b jeweils in zwei Kanäle 13Ia, 13IIa und 13Ib und 13IIb aufteilen. Die Kanäle 13Ia und 13IIa erstrecken sich bis zum Prothesenschaft 7e und sind mit jeweils einem der zweiten Kontaktflächen 12 elektrisch leitend verbunden. Ein weiterer elektrisch leitender Kanal 13IIIa verbindet die Kanäle 13Ia und 13IIa elektrisch leitend mit der dritten Kontaktfläche 12, die weder mit dem Kanal 13Ia noch mit dem Kanal 13IIa elektrisch leitend verbunden ist. Die Kanäle 13Ib und 13IIb erstrecken sich ebenfalls bis zum Prothesenschaft 7e und sind ebenfalls mit jeweils einem der zweiten Kontaktflächen 12 elektrisch leitend verbunden. Ein weiterer elektrisch leitender Kanal 13IIIb verbindet die Kanäle 13Ib und 13IIb elektrisch leitend mit der dritten Kontaktfläche 12, die weder mit dem Kanal 13Ib noch mit dem Kanal 13IIb elektrisch leitend verbunden ist.

Figur 14 zeigt eine erfindungsgemäße Unterarmprothese 1 wie in Figur 7 gemäß einem zweiten Ausführungsbeispiels. Wie die Prothesen in den Figuren 8 und 9 weist die Prothese 1 in Figur 14 im Bereich der Daumen- und Fingerdruckflächen 54a und 54b erste Kontaktflächen 16a und 16b auf, die jeweils mit einem elektrisch leitenden Kanal 13a bzw. 13b elektrisch leitend verbunden sind. Die Kanäle 13a und 13b erstrecken sich jeweils bis zu einer Mündung 14 im Handteil 4 der Prothese 1. In der Mündung 14 münden die Kanäle 13a und 13b in den elektrisch leitenden Kanal 13, welcher sich bis zu einem Verzweigungspunkt 15 im Bereich des gedachten Ellenbogengelenks befindet. Im Verzweigungspunkt 15 teilt sich der Kanal 13 in die elektrisch leitenden Kanäle 13I, 13II und 13III auf, welche sich bis zum Prothesenschaft 7f erstrecken. Im der Hand zugewandten Bereich des Prothesenschafts 7f sind drei zweite Kontaktflächen 12 in etwa maximal beabstandet zueinander angeordnet. Die zweiten Kontaktflächen 12 sind mit jeweils einem der Kanäle 13I, 13II und 13III elektrisch leitend verbunden. Die zweiten Kontaktflächen 12 sind ferner derart am Prothesenschaft 7f angeordnet, dass diese während des Tragens der Prothese 1 in elektrisch leitfähigem Kontakt mit einem Oberarmstumpf stehen, Somit besteht während des Tragens der Prothese 1 zwischen den ersten Kontaktflächen 16a und 16b und einem Oberarmstumpf über die Kanäle 13a bzw. 13b, 13 und 13I, 13II, 13III und die zweiten Kontaktflächen 12 ein elektrischer Kontakt.

Figur 15 zeigt einen Prothesenüberzug 100 für eine Fingerprothese gemäß einem dritten Ausführungsbeispiel. Äußerlich gleicht der Prothesenüberzug 100 einem menschlichen Finger 5. Im Inneren des Prothesenüberzugs 100 erstreckt sich entlang des ersten Fingergliedes 51, des zweiten Fingergliedes 52 sowie bis zum mittleren Bereich des Fingerendgliedes 53 ein Hohlraum 17. Der Prothesenüberzug weist im der gedachten Hand zugewandten Bereich des ersten Fingergliedes 51 eine Öffnung 18 zum Hohlraum 17 auf, über welcher der Prothesenüberzug 100 auf eine Fingerprothese aufziehbar ist. Während des Tragens des Prothesenüberzugs nimmt der Hohlraum 17 die Fingerprothese formschlüssig auf.

Ähnlich wie bei der Fingerprothese in Figur 4 ist am Fingerendglied 53 des Prothesenüberzugs 100 eine Eingabeeinheit zur Bedienung eines kapazitiv berührungsempfindlichen Eingabegerätes angeordnet, die im Bereich der Fingerdruckfläche 54 eine Kontaktfläche 11 aufweist. Die Kontaktfläche 11 ist auf ihrer dem Finger 5 abgewandten Seite verjüngend oder halbkreisförmig geformt.

## Patentansprüche

1. Prothese (1) oder Prothesenüberzug (100),
wobei die Prothese (1) oder der Prothesenüberzug (100) mindestens einen Bereich (16) aufweist, der zur Bedienung eines kapazitiven Eingabegerätes, insbesondere eines kapazitiv berührungsempfindlichen Eingabegerätes, geeignet ist, die Prothese (1) oder der Prothesenüberzug (100) mindestens eine erste Schicht (16, 12) aufweist, wobei diese Schicht (16, 12) als Bereich (16) zur Bedienung des kapazitiven Eingabegerätes eine erste Kontaktstelle (16), die mit dem Eingabegerät in Kontakt bringbar ist, und eine mit der ersten Kontaktstelle (16) elektrisch leitend verbundene zweite Kontaktstelle (12), die ihrerseits mit einem Körperteil elektrisch leitend verbindbar ist, aufweist,
**dadurch gekennzeichnet, dass**
die Prothese (1) oder der Prothesenüberzug (100) mindestens eine innere Schicht (16, 12) als erste Schicht (16, 12) und mindestens eine zweite, äußere Schicht (2, 3, 4) aufweist, wobei die zweite Schicht (2, 3, 4) die erste Schicht (16, 12) bis auf die Kontaktstellen (16, 12) umgibt.

2. Prothese (1) oder Prothesenüberzug (100) nach dem vorhergehenden Anspruch, **dadurch gekennzeichnet, dass** die zweite Kontaktstelle (12) eine Fläche von 1 bis 100 mm², vorteilhafterweise von 5 bis 50 mm², vorteilhafterweise von 20 bis 40 mm², vorteilhafterweise von 30 mm² aufweist.

3. Prothese (1) oder Prothesenüberzug (100) nach einem der Ansprüche 1 oder 2, **dadurch gekennzeichnet, dass** die erste (16) und/oder die zweite Kontaktstelle (12) leitfähiges Silikon enthalten oder daraus bestehen.

4. Prothese (1) oder Prothesenüberzug (100) nach einem der drei vorhergehenden Ansprüche, **dadurch gekennzeichnet, dass** die erste (16) und die zweite Kontaktstelle (12) über einen leitfähigen Träger (13), der Silikon, Polyurethan, Metalle, leitfähiges Garn, ein leitfähiges Kabel, eine leitfähige Flüssigkeit, oder andere leitfähige Materialien oder Kombinationen hiervon enthält oder daraus besteht, elektrisch leitend miteinander verbunden sind.

5. Prothese (1) oder Prothesenüberzug (100) nach dem vorhergehenden Anspruch, **dadurch gekennzeichnet, dass** der leitfähige Träger (13) Bestandteil der ersten Schicht (16, 12) ist.

6. Prothese (1) oder Prothesenüberzug (100) nach dem vorhergehenden Anspruch, **dadurch gekennzeichnet, dass** die zweite Schicht (2, 3, 4) elektrisch nichtleitend ist.

7. Prothese (1) oder Prothesenüberzug (100) nach einem der beiden vorhergehenden Ansprüche, **dadurch gekennzeichnet, dass** die innere Schicht (16, 12) in die äußere Schicht (2, 3, 4) einvulkanisiert und/oder eingeklebt ist.

8. Prothese (1) oder Prothesenüberzug (100) nach einem der beiden vorhergehenden Ansprüche, **dadurch gekennzeichnet, dass** die zweite Schicht (2, 3, 4) ein der Haut in Konsistenz und/oder Farbe ähnelndes Material enthält.

9. Prothese (1) oder Prothesenüberzug (100) nach einem der vorhergehenden Ansprüche, **dadurch gekennzeichnet, dass** die innere Schicht (16, 12) und/oder die äußere Schicht (2, 3, 4) Silikon enthält oder zu über 90% daraus besteht.

10. Prothese (1) oder Prothesenüberzug (100) nach einem der vorhergehenden Ansprüche, **dadurch gekennzeichnet, dass** der Bereich (16) zur Bedienung des kapazitiven Eingabegerätes eine Fläche von 1 bis 100 mm², vorteilhafterweise von 5 bis 50 mm², vorteilhafterweise von 20 bis 30 mm², vorteilhafterweise von 25 mm² aufweist.

11. Prothese (1) oder Prothesenüberzug (100) nach einem der vorhergehenden Ansprüche, **dadurch gekennzeichnet, dass** der Bereich (16) zur Bedienung des kapazitiven Eingabegerätes mit einem dünnen, gegebenenfalls farbadaptierten, Material zum Schutz des Bereichs (16) überzogen ist.

12. Prothese (1) oder Prothesenüberzug (100) nach einem der vorhergehenden Ansprüche, **dadurch gekennzeichnet, dass** die Prothese (1) oder der Prothesenüberzug (100) hochtemperaturvernetztes Silikon als Basismaterial enthält oder daraus besteht.

13. Prothese (1) oder Prothesenüberzug (100) nach einem der vorhergehenden Ansprüche, **dadurch gekennzeichnet, dass** die Prothese (1) oder der Prothesenüberzug (100) zur Bedienung eines Touchscreens, Touchdisplays, Touchpads, einer Touchmaus und/oder einer Multi-Touch-Oberfläche geeignet ist.

14. Prothese (1) oder Prothesenüberzug (100) nach einem der vorhergehenden Ansprüche, **dadurch gekennzeichnet, dass** die Prothese (1) oder der Prothesenüberzug (100) als Finger-, Hand-, Partialhand-, Arm-, Fuß-, Bein- oder Zehenprothese bzw. als Überzug für eine Finger-, Hand-, Partialhand-, Arm-, Fuß-, Bein-, Zehenprothese oder eine Arbeitsprothesenhand, wie z.B. eine Hook-Prothese, ausgebildet ist, wobei die zweite Kontaktstelle (12) mit einem Finger-, Hand-, Arm-, Bein-, Fuß- oder Zehenstumpf elektrisch leitend verbunden ist.

## Claims

1. Prosthesis (1) or prosthesis covering (100),
the prosthesis (1) or the prosthesis covering (100) having at least one region (16) which is suitable for operation of a capacitive input device, in particular a capacitive touch-sensitive input device, the prosthesis (1) or the prosthesis covering (100) having at least one first layer (16, 12), this layer (16, 12) having, as region (16) for operation of the capacitive input device, a first contact position (16) which can be brought into contact with the input device, and a second contact position (12) which is connected electrically conductively to the first contact position (16), said second contact position (12), for its part, being connectible electrically conductively to a body part,
**characterised in that**
the prosthesis (1) or the prosthesis covering (100) has at least one inner layer (16, 12) as first layer (16, 12) and at least one second, outer layer (2, 3, 4), the second layer (2, 3, 4) surrounding the first layer (16, 12) apart from the contact positions (16, 12).

2. Prosthesis (1) or prosthesis covering (100) according to the preceding claim, **characterised in that** the second contact position (12) has a surface area of 1 to 100 mm², advantageously of 5 to 50 mm², advantageously of 20 to 40 mm², advantageously of 30 mm².

3. Prosthesis (1) or prosthesis covering (100) according to one of the claims 1 or 2, **characterised in that** the first (16) and/or the second contact position (12) comprise conductive silicone or consist thereof.

4. Prosthesis (1) or prosthesis covering (100) according to one of the three preceding claims, **characterised in that** the first (16) and the second contact position (12) are connected electrically conductively to each other via a conductive carrier (13) which comprises silicone, polyurethane, metals, conductive yarn, a conductive cable, a conductive liquid or other conductive materials or combinations hereof or consists thereof.

5. Prosthesis (1) or prosthesis covering (100) according to the preceding claim, **characterised in that** the conductive carrier (13) is a component of the first layer (16, 12).

6. Prosthesis (1) or prosthesis covering (100) according to the preceding claim, **characterised in that** the second layer (2, 3, 4) is electrically non-conductive.

7. Prosthesis (1) or prosthesis covering (100) according to one of the two preceding claims, **characterised in that** the inner layer (16, 12) is vulcanised and/or glued into the outer layer (2, 3, 4).

8. Prosthesis (1) or prosthesis covering (100) according to one of the two preceding claims, **characterised in that** the second layer (2, 3, 4) comprises a material which resembles the skin in consistency and/or colour.

9. Prosthesis (1) or prosthesis covering (100) according to one of the preceding claims, **characterised in that** the inner layer (16, 12) and/or the outer layer (2, 3, 4) comprises silicone or consists up to over 90% thereof.

10. Prosthesis (1) or prosthesis covering (100) according to one of the preceding claims, **characterised in that** the region (16) for operation of the capacitive input device has a surface area of 1 to 100 mm², advantageously of 5 to 50 mm², advantageously of 20 to 30 mm², advantageously of 25 mm².

11. Prosthesis (1) or prosthesis covering (100) according to one of the preceding claims, **characterised in that** the region (16) for operation of the capacitive input device is covered with a thin, possibly colour-adapted, material for protection of the region (16).

12. Prosthesis (1) or prosthesis covering (100) according to one of the preceding claims, **characterised in that** the prosthesis (1) or the prosthesis covering (100) comprises high-temperature-crosslinked silicone as basic material or consists thereof.

13. Prosthesis (1) or prosthesis covering (100) according to one of the preceding claims, **characterised in that** the prosthesis (1) or the prosthesis covering (100) is suitable for operation of a touch screen, touch display, touch pad, a touch mouse and/or a multi-touch surface.

14. Prosthesis (1) or prosthesis covering (100) according to one of the preceding claims, **characterised in that** the prosthesis (1) or the prosthesis covering (100) is configured as finger-, hand-, partial hand-, arm-, foot-, leg- or toe prosthesis or as covering for a
finger-, hand-, partial hand-, arm-, foot-, leg-, toe prosthesis or a working prosthetic hand, such as e.g. a hook prosthesis, the second contact position (12) being connected electrically conductively to a finger-, hand-, arm-, leg-, foot- or toe stump.

## Revendications

1. Prothèse (1) ou revêtement de prothèse (100),
la prothèse (1) ou le revêtement de prothèse (100) comprenant au moins une zone (16) qui est conçue pour la commande d'un appareil d'entrée capacitif, plus particulièrement d'un appareil d'entrée capacitif sensible au contact, la prothèse (1) ou le revêtement de prothèse (100) comprenant au moins une première couche (16, 12), cette couche (16, 12) comprenant, en tant que zone (16) pour la commande de l'appareil d'entrée capacitif, un premier point de contact (16), qui peut être mis en contact avec l'appareil d'entrée, et un deuxième point de contact (12), relié de manière électro-conductrice au premier point de contact (16), qui peut être relié, de son côté, de manière électro-conductrice avec une partie du corps,
**caractérisé en ce que**
la prothèse (1) ou le revêtement de prothèse (100) comprend au moins une couche interne (16, 12) en tant que première couche (16, 12) et au moins une deuxième couche externe (2, 3, 4), la deuxième couche (2, 3, 4) entourant la première couche (16, 12) à l'exception des points de contact (16, 12).

2. Prothèse (1) ou revêtement de prothèse (100) selon la revendication précédente, **caractérisé en ce que** le deuxième point de contact (12) présente une surface de 1 à 100 mm², de manière avantageuse de 5 à 50 mm², de manière avantageuse de 20 à 40 mm², de manière avantageuse de 30 mm².

3. Prothèse (1) ou revêtement de prothèse (100) selon l'une des revendications 1 ou 2, **caractérisé en ce que** le premier (16) et/ou le deuxième point de contact (12) contiennent du silicone conducteur ou en sont constitués.

4. Prothèse (1) ou revêtement de prothèse (100) selon l'une des trois revendications précédentes, **caractérisé en ce que** le premier (16) et le deuxième point de contact (12) sont reliés de manière électro-conductrice entre eux par l'intermédiaire d'un support conducteur (13), qui contient ou est constitué de silicone, de polyuréthane, de métaux, d'un fil conducteur, d'un câble conducteur, d'un liquide conducteur ou d'autres matériaux conducteurs ou des combinaisons de ceux-ci.

5. Prothèse (1) ou revêtement de prothèse (100) selon la revendication précédente, **caractérisé en ce que** le support conducteur (13) fait partie de la première couche (16, 12).

6. Prothèse (1) ou revêtement de prothèse (100) selon la revendication précédente, **caractérisé en ce que** la deuxième couche (2, 3, 4) n'est pas électro-conductrice.

7. Prothèse (1) ou revêtement de prothèse (100) selon l'une des deux revendications précédentes, **caractérisé en ce que** la couche interne (16, 12) est vulcanisée et/ou collée dans la couche externe (2, 3, 4).

8. Prothèse (1) ou revêtement de prothèse (100) selon l'une des deux revendications précédentes, **caractérisé en ce que** la deuxième couche (2, 3, 4) contient un matériau similaire à la peau en ce qui concerne la consistance et/ou la couleur.

9. Prothèse (1) ou revêtement de prothèse (100) selon l'une des revendications précédentes, **caractérisé en ce que** la couche interne (16, 12) et/ou la couche externe (2, 3, 4) contient du silicone ou en est constitué à plus de 90 %.

10. Prothèse (1) ou revêtement de prothèse (100) selon l'une des revendications précédentes, **caractérisé en ce que** la zone (16) pour la commande de l'appareil d'entrée capacitif présente une surface de 1 à 100 mm², de manière avantageuse de 5 à 50 mm², de manière avantageuse de 20 à 30 mm², de manière avantageuse de 25 mm².

11. Prothèse (1) ou revêtement de prothèse (100) selon l'une des revendications précédentes, **caractérisé en ce que** la zone (16) pour la commande de l'appareil d'entrée capacitif est revêtue d'un matériau mince, le cas échéant adapté en termes de couleur, pour la protection de la zone (16).

12. Prothèse (1) ou revêtement de prothèse (100) selon l'une des revendications précédentes, **caractérisé en ce que** la prothèse (1) ou le revêtement de prothèse (100) contient un silicone réticulé à haute température en tant que matériau de base ou en est constitué.

13. Prothèse (1) ou revêtement de prothèse (100) selon l'une des revendications précédentes, **caractérisé en ce que** la prothèse (1) ou le revêtement de prothèse (100) est conçu pour la commande d'un écran tactile, d'un affichage tactile, d'une tablette tactile, d'une souris tactile et/ou d'une surface multi-tactile.

14. Prothèse (1) ou revêtement de prothèse (100) selon l'une des revendications précédentes, **caractérisé en ce que** la prothèse (1) ou le revêtement de prothèse (100) est conçu comme une prothèse de doigt, de main, de main partielle, de bras, de pied, de jambe ou d'orteil ou comme un revêtement pour une prothèse de doigt, de main, de main partielle, de bras, de pied, de jambe ou d'orteil ou une main de prothèse de travail, par exemple une prothèse à crochet, le deuxième point de contact (12) étant relié de manière électro-conductrice avec un moignon de doigt, de main, de bras, de jambe, de pied ou d'orteil.
